# EUROPEAN PATENT APPLICATION

(11) **EP 4 052 694 A1**
(43) Date of publication of application: **07.09.2022**
(21) Application number: 20888845.3
(22) Date of filing: 20.11.2020
(51) Int. Cl.: A61K 9/00, A61K 9/08, A61K 47/10, A61K 47/12, A61K 47/40, A61K 31/4439, A61P 27/02

(54) **EYE DROP COMPOSITION FOR PREVENTING OR TREATING EYE DISEASE**

(30) Priority: 21.11.2019 KR 20190150781
(71) Applicant: Samjin Pharmaceutical Co., Ltd., Seoul 04054 (KR); Aptabio Therapeutics Inc., Yongin-si, Gyeonggi-do 16954 (KR)
(72) Inventor: KI, Min-hyo, Seongnam-si Gyeonggi-do 13595 (KR); KIM, Jin-cheul, Seoul 07216 (KR); CHOI, Mee-Hwa, Yongin-si Gyeonggi-do 16874 (KR); LEE, Dong-Ha, Suwon-si Gyeonggi-do 16550 (KR); MOON, Sung Hwan, Suwon-si Gyeonggi-do 16222 (KR); LEE, Soo Jin, Suwon-si Gyeonggi-do 16709 (KR)
(74) Representative: Gille Hrabal Partnerschaftsgesellschaft mbB Patentanwälte
(86) International application number: PCT/KR2020/016538
(87) International publication number: WO 2021/101344

(57) **Abstract**

The present invention relates to an ophthalmic preparation containing an active ingredient selected from 3-phenyl-4-propyl-1-(pyridin-2-yl)-1H-pyrazol-5-ol compound or a pharmaceutically acceptable salt thereof. The eye drop according to the present invention is excellent in stability and safety and shows an excellent effect on prevention or treatment of eye diseases in such a way that an active ingredient thereof reaches a posterior segment of an eyeball simply through instillation rather than a direct injection into an eyeball.

## Description

### Technical Field

The present invention relates to an ophthalmic preparation containing an active ingredient selected from 3-phenyl-4-propyl-1-(pyridin-2-yl)-1H-pyrazol-5-ol or a pharmaceutically acceptable salt thereof. The eye drop according to the present invention is excellent in storage stability and safety and shows an excellent effect on prevention or treatment of eye diseases in such a way that an active ingredient thereof reaches not only an anterior segment of an eyeball but also a posterior segment of the eyeball through instillation alone rather than a direct injection into the eyeball for the prevention or treatment of eye diseases.

### Background

An eye disease intended by the present invention refers to a disease which affects an eye or a part of the eye or a specific area of the eye or is associated with a specific area of the eye. The eye includes an eyeball, the tissues and body fluids that constitute the eyeball, muscles around the eye, and a portion of optic nerves within or adjacent to the eyeball. Anterior segment eye disease is a disease which affects or involves an anterior region and area such as muscles around the eye, an eyelid, or eyeball tissues or body fluids, which are located on a posterior wall of a lens capsule or in front of a ciliary muscle. In other words, anterior segment eye disease primarily affects or involves a conjunctiva, a cornea, an anterior chamber of eye, an iris, a posterior chamber of eye, a crystalline lens or a lens capsule, and blood vessels and nerves passing an anterior area or region. And, posterior segment eye disease is a disease which primarily affects or involves a posterior area and region such as a choroid or a sclera, a vitreous body, a vitreous chamber, a retina, optic nerves, and vessels and nerves passing a posterior region or area. Thus, posterior segment eye diseases may include macular degeneration (age-related macular degeneration (AMD) such as non-exudative senile macular degeneration and exudative senile macular degeneration); choroidal neovascularization; acute macular neuroretinopathy; macular edema (cystoid macular edema and diabetic macular edema); Behcet's disease, retinal disorder, diabetic retinopathy (including proliferative diabetic retinopathy); retinal arterial occlusive disease; retinal vein occlusion; uvea retinopathy; retinal detachment; posterior segmental trauma; posterior segment eye disease caused or affected by ocular laser treatment; posterior segment eye disease caused or affected by photodynamic therapy or photocoagulation, radiation retinopathy, epiretinal membrane disorder, branch retinal vein occlusion, anterior ischemic optic neuropathy, non-retinopathy diabetic retinal dysfunction, retinitis pigmentosa, glaucoma, and posterior segment eye disease caused or affected by glaucoma. In the present specification, the disease caused or affected by glaucoma may be defined as a concept, in which the disease is included in glaucoma.

Angiogenesis, which is included in a main control mechanism by the composition of the present invention, refers to a process in which a new blood vessel sprouts from an existing microvessel, and the sprouting blood vessel proliferates to produce a new capillary blood vessel. Angiogenesis is a highly regulatory process that occurs not only due to various pro-angiogenic stimuli such as growth factors, cytokines and other physiological molecules, but also in response to other factors such as hypoxia and low pH. Angiogenesis is a normal process which is very important in the development of embryos in the human body, fetal growth, placental proliferation, luteinization, tissue regeneration and wound healing. However, there are diseases which are caused by angiogenesis itself, if angiogenesis is abnormally increased or is not normally controlled. Representative diseases related to this vascularization include eye diseases such as diabetic retinopathy, retinopathy of prematurity (ROP), age-related macular degeneration, corneal neovascularization, neovascular glaucoma, and degeneration of spots, pterygium, retinitis pigmentosa, granular conjunctivitis and the like. In the eyeball, a vascularization mechanism needs to be suppressed. However, if being activated due to a wrong signal, the vascularization mechanism causes a serious eye disease, which also becomes a major cause of acquired blindness. In particular, the retina is an organ that rapidly reacts to active oxygen by consuming a higher amount of oxygen than other muscles, and it is reported that a high concentration of glucose promotes a VEGF expression through activation of active oxygen, thereby inducing the progression and acceleration of eyeball injuries. Therapy for eye diseases associated with vascularization includes surgical treatments such as laser treatment, photocoagulation, cryocoagulation, photodynamic therapy and the like, but there is a large limitation that this surgical procedure cannot be applied to all patients, and it is also known that a success rate thereof is low.

The eyeball is composed of a variety of complex tissues, and has a limit to the delivery of pharmacologically active materials into the eyeball tissues in case of instilling active ingredients due to various factors that interfere with drug delivery between the tissues. For this reason, besides a therapeutic agent for diseases associated with cornea and conjunctiva, the commercially available ophthalmic products for eye diseases are very limited. In particular, in case of drugs used for the treatment of posterior segment eye diseases, intravitreal injection is mainly performed, but has a limit to a single dose, and shows a very low patient's compliance due to repeated injections, as well as a very high risk of bleeding, pain, infection, retinal detachment, etc. For this reason, with regard to posterior segment eye diseases, the patients' compliance is remarkably low, and a burden on treatment costs is very high. Thus, there is an urgent need for developing a novel type of composition capable of delivering a drug to target tissues even with simple instillation rather than injection, and showing a therapeutic effect.

### Detailed Description of the Invention

### Technical Problem

The present invention relates to an ophthalmic preparation containing an active ingredient selected from 3-phenyl-4-propyl-1-(pyridin-2-yl)-1H-pyrazol-5-ol or a pharmaceutically acceptable salt thereof. The eye drop according to the present invention has satisfactory stability without a change in quality over time because the stability is good and can be useful as a therapeutic agent for posterior segment eye diseases with a high patient's compliance with medication in such a way that an active ingredient thereof reaches not only an anterior segment of an eyeball but also a target region including a posterior segment of the eyeball even through instillation rather than direct injection into the eyeball for treatment of eye diseases.

Patent Document 1 (Korean Registered Patent No. 10-1280160) describes a composition for preventing and treating osteoporosis, and Patent Document 2 (Korean Registered Patent No. 10-1633957) describes a composition for preventing or treating kidney disease, in which a pyrazole-based compound of the present invention is described as an active ingredient. However, the possibility of using the pyrazole-based compound (3-phenyl-4-propyl-1-(pyridin-2-yl)-1H-pyrazol-5-ol) as an eye drop is not mentioned at all. Also, there is no example of using an eye drop for the treatment of osteoporosis and kidney disease, which is specified in prior documents. According to the present invention, it has been confirmed that an ophthalmic composition according to one embodiment can be used as an eye drop for preventing or treating eye diseases by efficiently inhibiting angiogenesis in the eyeball. Accordingly, it has been also confirmed that the ophthalmic composition according to the present invention can be applied to the treatment of eye diseases in addition to the uses specified in the prior documents.

Although all the existing therapeutic agents for retinal diseases have serious side effects, intraocular injection has been adopted because drugs are difficult to reach the retina of the posterior segment through instillation. In the present invention, a liquid ophthalmic composition, which secures the stability and safety of the 3-phenyl-4-propyl-1-(pyridin-2-yl)-1H-pyrazol-5-ol compound, has been developed to finally show that the composition of the present invention may have a more excellent effect on retinal treatment simply through instillation than that of the existing therapeutic agents through intraocular injection according to an animal test. In conclusion, there has been no case of securing the stability and safety of the compound of the present invention (3-phenyl-4-propyl-1-(pyridin-2-yl)-1H-pyrazol-5-ol), delivering the present compound into the posterior segment through instillation, and studying an effect of the present compound on animal treatment through an in vivo evaluation, and this case has been first demonstrated through the present invention.

### Technical Solution

A method for achieving the objective of the present invention is as follows. All combinations of various elements disclosed in the present invention belong to the scope of the present invention, but it cannot be considered that the scope of the present invention is limited by the specific description described below.

The present invention may provide an eye drop for preventing or treating eye diseases, which comprises3-phenyl-4-propyl-1-(pyridin-2-yl)-1H-pyrazol-5-ol represented by a following formula 1 or a pharmaceutically acceptable salt thereof as an active ingredient, as well as a solubilizing agent and a buffer agent, in which a pH range is 3.5 to 8.5. In particular, said eye diseases may be posterior segment eye diseases. For example, the present invention may provide an eye drop for treating eye diseases or posterior segment eye diseases, which comprises 3-phenyl-4-propyl-1-(pyridin-2-yl)-1H-pyrazol-5-ol [Formula 1] or a pharmaceutically acceptable salt thereof as an active ingredient by 0.1 w/v% to 2.0 w/v% as an active ingredient, as well as a solubilizing agent and a buffer agent, in which a pH range is 3.5 to 8.5 or 4.5 to 8.5.

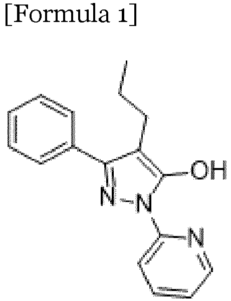

According to one embodiment, the eye drop may comprise 3-phenyl-4-propyl-1-(pyridin-2-yl)-1H-pyrazol-5-ol or a pharmaceutically acceptable salt thereof by 0.1 w/v% or more and 2.0 w/v% or less as an active ingredient of the present invention. According to one embodiment, the eye drop may comprise the active ingredient by 0.1 w/v% or more and 2.0 w/v% or less. Thus, even through a direct instillation into the eyeball, the active ingredient may be sufficiently dissolved to show a sufficient effect and achieve excellent stability and safety. Hereinafter, in the present specification, the "active ingredient" may refer to "3-phenyl-4-propyl-1-(pyridin-2-yl)-1H-pyrazol-5-ol or a pharmaceutically acceptable salt thereof," unless otherwise specified.

In the present invention, pharmaceutically acceptable salts may refer to the salts conventionally used in a pharmaceutical industry, for example, inorganic ion salts prepared from calcium, potassium, sodium, magnesium and the like; inorganic acid salts prepared from hydrochloric acid, nitric acid, phosphoric acid, bromic acid, iodic acid, perchloric acid, tartaric acid, sulfuric acid and the like; organic acid salts prepared from acetic acid, trifluoroacetic acid, citric acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbric acid, carbonic acid, vanillic acid, hydroiodic acid, etc.; sulfonic acid salts prepared from methanesulfonic acid, ethanesulfonic acid, benzenesulfonic acid, p-toluenesulfonic acid, naphthalenesulfonic acid and the like; amino acid salts prepared from glycine, arginine, lysine, etc.; amine salts prepared from trimethylamine, triethylamine, ammonia, pyridine, picoline, etc.; and the like, but types of salts meant in the present invention are not limited to those listed salts. For example, in one embodiment, the pharmaceutically acceptable salt may be hydrochloride.

Unlike an orally delivered composition, the composition of the present invention may be an ophthalmic composition applied to the eyeball, and thus may need to be provided in a pH range that does not cause any corneal and conjunctival damage with less foreign body sensation and irritation, and may need to show a valid effect on treatment of eye ball tissues in a posterior segment, which is intended by the present invention. In addition, 3-phenyl-4-propyl-1-(pyridin-2-yl)-1H-pyrazol-5-ol included in the ophthalmic composition of the present invention may be an active material, of which stability varies depending on pH, and thus may be prepared with a pH of 3.5 to 8.5, for example, pH of 4.5 to 8.5, in order to enhance chemical stability of the drug, minimize eyeball irritation, and deliver the drug to the posterior segment.

According to one embodiment, the eye drop may comprise a solubilizing agent. In one embodiment, the solubilizing agent may be used to increase the solubility of 3-phenyl-4-propyl-1-(pyridin-2-yl)-1H-pyrazol-5-ol, and may comprise at least one selected from the group consisting of surfactants, solvents and mixtures thereof. In one embodiment, the eye drop may comprise at least one of surfactants and at least one of solvents. In one embodiment, the solubilizing agent may be at least one selected from the group consisting of surfactants, solvents, and mixtures thereof.

In one embodiment, the eye drop may comprise the above-described solubilizing agent, so that an active ingredient thereof may be well dissolved and may not be precipitated even after long-term storage, thus resulting in better physical stability and chemical stability. In one embodiment, a concentration of the solubilizing agent in the eye drop maybe 0.1 w/v% to 25.0 w/v%. In one embodiment, the type of the solubilizing agent may not be particularly limited, but may include, for example, at least one selected from the group consisting of surfactants, solvents, and mixtures thereof as follows. The solubility of 3-phenyl-4-propyl-1-(pyridin-2-yl)-1H-pyrazol-5-ol, which is an active material of the present invention, in water may be measured to be about 3.4 µg/mL. Thus, in one embodiment, the eye drop may comprise the solubilizing agent to dissolve 3-phenyl-4-propyl-1-(pyridin-2-yl)-1H-pyrazol-5-ol by 0.1% (1,000 µg/mL) to 2.0% (20,000 µg/mL).

Out of the solubilizing agents of the present invention, the surfactant may be an additive used for dissolving the active ingredient by lowering an interfacial tension between the active ingredient and an aqueous solution when 3-phenyl-4-propyl-1-(pyridin-2-yl)-1H-pyrazol-5-ol is dissolved in water or is mixed with the solvent. And, this surfactant may be a material to stabilize the active ingredient so that the active ingredient may be well dissolved and may not be precipitated while the active ingredient is in a state of an aqueous solution or being mixed with a solvent.

In the eye drop of one embodiment, the type of the surfactant may not be particularly limited as long as it may stabilize the active ingredient of one embodiment, and those skilled in the art may select and use a suitable one of known surfactants.

For example, the eye drop of one embodiment may comprise at least one selected from the group consisting of nonionic surfactants, ionic surfactants, and mixtures thereof as a surfactant.

For example, the eye drop of one embodiment may comprise as the nonionic surfactant, at least one selected from the group consisting of polyoxyethylene sorbitan fatty acid ester (Polysorbates), polyoxyethylene fatty acid ester (Myrj), sorbitan ester (Span), glycerol monostearate, nonoxynol, octoxynol, polyethylene glycol, polypropylene glycol, polyoxyethylene-polyoxypropylene copolymer (Poloxamer), polyoxyethylene castor oil, polyoxyethylene hydrogenated castor oil (Cremophor), cyclodextrin, and hydroxypropylbetadex, but the embodiment is not limited thereto.

For example, the eye drop of one embodiment may comprise as the surfactant at least one selected from the group consisting of polyoxyethylene sorbitan fatty acid ester, polyoxyethylene fatty acid, polyoxyethylene-polyoxypropylene copolymer, polyoxyethylene hydrogenated castor oil, hydroxypropylbetadex, and mixtures thereof, but is not limited thereto.

For example, the eye drop of one embodiment may comprise anionic, cationic and amphoteric ionic surfactants as the ionic surfactant.

The eye drop of one embodiment may comprise, for example, as the anionic surfactant at least one selected from the group consisting of sulfates (alkyl sulfates, alkyl ether sulfates), sulfonates (docusates, alkyl benzene sulfonates), carboxylates (alkyl carboxylates-fatty acid salts, carboxylate fluoro surfactants), and phosphates (alkyl aryl ether phosphates, alkyl ether phosphates), but is not limited thereto.

For example, the eye drop of one embodiment may comprise sodium lauryl sulfate, which is one example of alkyl sulfate, as the anionic surfactant. For example, the eye drop of one embodiment may comprise as the cationic surfactant at least one selected from the group consisting of quaternary ammonium and pyridinium cationic surfactants, but is not limited thereto, and may comprise benzalkonium hydrochloride as one example.

The eye drop of one embodiment may comprise at least one selected from phospholipids as the amphoteric surfactant, but is not limited thereto, and may comprise lecithin as one example.

Out of the solubilizing agents of the present invention, the solvent may refer to an additive capable of dissolving the active ingredient in a liquid state to form a homogeneously dispersed mixture (solution) in a molecular or ionic state, and may refer to a material for increasing the dissolution of 3-phenyl-4-propyl-1-(pyridin-2-yl)-1H-pyrazol-5-ol along with the surfactant in a waterless or aqueous solution state.

In the eye drop of one embodiment, the type of the solvent may not be particularly limited as long as the solvent may sufficiently dissolve the active ingredient of one embodiment, and those skilled in the art may select and use a suitable one for the present invention among known solvents.

For example, the eye drop of one embodiment may comprise as the solvent at least one selected from water-soluble glycols such as ethylene glycol, diethylene glycol, triethylene glycol, propylene glycol, polypropylene glycol, etc.; esters of the water-soluble glycols such as propylene glycol diacetate, etc.; natural, synthetic or semisynthetic oils such as castor oil, lanolin oil, mineral oil, peanut oil, lanolin oil, etc.; medium-chain fatty acid (capric and caprylic) mono-diglycerin (Campmul MCM) such as Capmul MCM NF; ethanol; phenylethyl alcohol; caprylic triglycerides; capric acid triglycerides; and caprylic capric acid triglycerides.

For example, in one embodiment, the solvent may be selected from the group consisting of castor oil, ethanol, propylene glycol, phenylethyl alcohol, propylene glycol diacetate, glycerin, medium-chain fatty acid (capric and caprylic) mono-diglycerin (Campmul MCM), and mixtures thereof, but is not limited thereto.

In the present invention, the buffer agent may refer to a pharmaceutical additive used for preventing a hydrogen ion exponent of a solution from being significantly changed due to a common ion effect even when an acid or a base is added. The active ingredient of the present invention, which is 3-phenyl-4-propyl-1-(pyridin-2-yl)-1H-pyrazol-5-ol, may refer to a material, which has a varying degree of causing precipitation and generating related substances, that is, the varying physicochemical stability, depending on pH. Thus, the buffer agent may be used in the composition of the present invention in order to maintain a pH in the range capable of stabilizing 3-phenyl-4-propyl-1-(pyridin-2-yl)-1H-pyrazol-5-ol.

In the eye drop of one embodiment, the type of the buffer agent may not be particularly limited as long as it may maintain a pH range capable of stabilizing the active ingredient of one embodiment, and those skilled in the art may select and use a suitable one for the present invention among known buffer agents.

For example, the buffer agent of one embodiment may be prepared and used in the form of a buffer solution with addition of a weak acid and a conjugate base thereof or a weak base and a conjugate acid thereof, and may be compounded and used in the form of citrate buffer solution, acetate buffer solution, phosphate buffer solution, borate buffer solution, tris salt buffer solution, etc., but is not limited thereto. Specifically, the citric acid buffer solution may be used by mixing citric acid and/or a pharmaceutically acceptable salt thereof with acetic acid, sodium hydroxide, disodium hydrogen phosphate, sodium monohydrogen phosphate, ammonium salt, etc., which may be used as a conjugate base of citric acid; the acetate (ammonium acetate) buffer solution may be prepared by using acetic acid and/or a pharmaceutically acceptable salt thereof such as ammonium acetate, potassium acetate, etc.; the phosphate buffer solution may be prepared by mixing phosphoric acid and/or a pharmaceutically acceptable salt thereof with sodium hydroxide, potassium hydroxide, etc.; the borate buffer solution may be prepared by mixing boric acid and/or a pharmaceutically acceptable salt thereof with sodium chloride, sodium hydroxide, potassium chloride, etc.; and the tris buffer solution may be prepared by mixing 2-amino-2-hydroxymethyl-1,3-propanediol and/or hydrochloride, acetate, etc. including sodium chloride, but is not limited thereto.

An acidifying agent or a basifying agent may be used to adjust the pH of the composition together with the buffer agent of the present invention, the acidifying agent used may include citric acid, hydrochloric acid, phosphoric acid, acetic acid, sulfuric acid, etc., and the basifying agent used may include sodium hydroxide, sodium carbonate, potassium carbonate, and the like, but is not limited thereto.

In addition, the eye drop of the present invention may further comprise at least one additive selected from the group consisting of a thickener and an isotonic agent.

If the eye drop of one embodiment comprises the thickener, the type of the thickener may not be particularly limited, and may include at least one selected from the group consisting of, for example, methylcellulose, hydroxymethylcellulose, hydroxyethylcellulose, hydroxypropylcellulose, hydroxypropylmethylcellulose, amorphous cellulose, polysaccharides including starch derivatives, polyvinyl alcohol and polyvinylpyrrolidone, and mixtures thereof.

The isotonic agent may be added in an amount enough to maintain an osmotic pressure of the eye drop similar to that of tear fluid, and may include chlorides including sodium chloride, and sugars including mannitol, but is not limited thereto.

The eye diseases intended by the present invention may include anterior segment eye diseases and posterior segment eye diseases, and for example, the posterior segment eye diseases may be selected. Anterior segment eye diseases may refer to diseases which affect or involve an anterior area and region such as muscles around the eye, eyelids, or eyeball tissues or body fluids, which are located on a posterior wall of a lens capsule or in front of a ciliary muscle. The anterior segment eye diseases may refer to at least one selected from the group consisting of dry eye, keratitis, conjunctivitis, scleritis, cataract, pinguecula, conjunctival nevus, ota nevus and corneal opacity (corneal tattooing), or at least one selected from the group consisting of other diseases associated with conjunctiva, cornea, anterior chamber of eye, iris, posterior chamber of eye, crystalline lens or lens capsule, and vessels and nerves passing the anterior area or region.

Posterior segment eye diseases may refer to at least one selected from the group consisting of diabetic retinopathy (DR), diabetic macular edema, age-related macular degeneration, acute macular neuroretinopathy, retinopathy of prematurity (ROP), polypoidal choroidal vasculopathy, ischemic proliferative retinopathy, retinitis pigmentosa, cone dystrophy, Behcet's disease, retinal disorders, proliferative vitreoretinopathy (PVR), retinal artery occlusion, retinal vein occlusion, retinitis, uveitis, Leber's hereditary optic neuropathy, retinal detachment, retinal pigment epithelial detachment, neovascular glaucoma, retinal neovascularization and choroidal neovascularization (CNV), trauma of the posterior segment, posterior segment eye diseases caused or affected by ocular laser treatment, posterior segment eye diseases caused or affected by photodynamic therapy or photocoagulation, radiation retinopathy, epiretinal membrane disorder, branch retinal vein occlusion, anterior ischemic optic nerve disorder, non-retinopathy diabetic retinal dysfunction, retinitis pigmentosa, glaucoma, and diseases caused or affected by glaucoma.

The eye drop containing 3-phenyl-4-propyl-1-(pyridin-2-yl)-1H-pyrazol-5-ol of the present invention was confirmed to show excellent physical stability and chemical stability (Test Example 1), and also confirmed to show excellent safety, thereby achieving excellent eye drop feeling (Test Example 2). In addition, it was confirmed from a non-clinical test that 3-phenyl-4-propyl-1-(pyridin-2-yl)-1H-pyrazol-5-ol is distributed up to the posterior segment of the eye simply through ophthalmic administration rather than injection (Test Examples 3 and 5). Furthermore, as a result of conducting an efficacy test with animals with induced CNV used for developing a therapeutic agent for macular degeneration and macular edema, which are representative posterior segment eye diseases, it was confirmed that there is an excellent therapeutic effect simply through instillation (see Test Examples 3 to 5).

Meanwhile, in the case of posterior segment eye diseases accompanied by blindness, such as macular degeneration and macular edema, an intraocular injection method has been used as the only treatment method. However, this intraocular injection fails to be periodically performed from the third year of treatment due to a very low patient's compliance with injection treatment. Thus, it is known that such failure of intraocular injection eventually leads to gradual blindness. In the case of applying the eye drop of 3-phenyl-4-propyl-1-(pyridin-2-yl)-1H-pyrazol-5-ol of the present invention for these diseases, it may be possible to address various side effects such as fear, pain, bleeding, inflammation, and blindness caused by the low patient's compliance with injection of the conventional therapeutic agent for posterior segment eye diseases, which needs to be directly injected into the eyeball. In addition, the eye drop according to one embodiment has secured advantages such as convenience, etc., in that the eye drop may be easily administered by the patients themselves, and thus this eye drop may be very useful for patients with eye diseases including posterior segment eye diseases.

According to a preferred embodiment aspect of the present invention, the composition of the present invention may provide an eye drop comprising the 3-phenyl-4-propyl-1-(pyridin-2-yl)-1H-pyrazol-5-ol compound with excellent physical and chemical stability and safety, and may also show excellent efficacy by delivering the 3-phenyl-4-propyl-1-(pyridin-2-yl)-1H-pyrazol-5-ol compound to the posterior segment, and thus may be used for treatment of not only anterior segment eye diseases but also posterior segment eye diseases without injection administration.

The ophthalmic composition may be prepared as a liquid eye drop, but embodiments are not limited thereto, and may be prepared into a dosage form of an ointment, a gelling agent, etc., according to a formulation method.

The present invention may provide a method for preventing or treating eye diseases including a step of administering the eye drop comprising 3-phenyl-4-propyl-1-(pyridin-2-yl)-1H-pyrazol-5-ol represented by formula 1 or a pharmaceutically acceptable salt thereof as an active ingredient; a solubilizing agent; and a buffer agent into a subject in need of treatment.

The present invention may provide a use of the eye drop for preventing or treating eye diseases, which comprises 3-phenyl-4-propyl-1-(pyridin-2-yl)-1H-pyrazol-5-ol represented by formula 1 or a pharmaceutically acceptable salt thereof as an active ingredient; a solubilizing agent; and a buffer agent.

The present invention may provide a use of the ophthalmic composition comprising 3-phenyl-4-propyl-1-(pyridin-2-yl)-1H-pyrazol-5-ol represented by formula 1 or a pharmaceutically acceptable salt thereof as an active ingredient; a solubilizing agent; and a buffer agent in order to prepare the eye drop for preventing or treating eye diseases.

The present invention may provide a method for preparing the eye drop, including a step of mixing 3-phenyl-4-propyl-1-(pyridin-2-yl)-1H-pyrazol-5-ol represented by formula 1 or a pharmaceutically acceptable salt thereof; a solubilizing agent; and a buffer agent. Said preparation method may further include a step of mixing other additives.

For the use of the eye drop according to the present invention, the use of the ophthalmic composition, the preparation method thereof, and the prevention or treatment method including the step of administering said eye drop, the description of the eye drop mentioned above may be equally applied if not contradictory to each other.

### Advantageous Effects

The present invention relates to an eye drop of 3-phenyl-4-propyl-1-(pyridin-2-yl)-1H-pyrazol-5-ol compound showing a valid effect on treatment of eye diseases, and said eye drop shows not only a high patient's compliance with medication but also excellent storage stability and safety, and thus can be useful as a therapeutic agent for eye diseases. The eye drop of the present invention can deliver a drug to a posterior segment through enhanced stabilization and safety of 3-phenyl-4-propyl-1-(pyridin-2-yl)-1H-pyrazol-5-ol so as to show an effect similar to or more excellent than an anti-VEGF drug in the existing form of intraocular injection, and thus is expected to provide a novel treatment option in addition to injection administration to patients even with posterior segment eye diseases such as macular degeneration.

### Brief Description of the Drawings

FIGS. 1 to 3 are graphs showing an amount of related substances produced depending on the pH of the ophthalmic composition measured according to Test Example 1.
FIG. 4 is a graph showing the result of observing and digitizing the size of CNV lesions after performing a retinal imaging evaluation using FFA after administration of the composition of the present invention (Example 13) and the control group according to Test Example 3.
FIG. 5 is a picture showing the comparison of the sizes of CNV lesions by performing a retinal imaging evaluation using FFA after administration of the composition of the present invention (Example 13) and the control group according to Test Example 3.
FIG. 6 is a graph showing the result of observing and digitizing the cross-sectional area of CNV lesions after performing a retinal imaging evaluation using OCT after administration of the composition of the present invention (Example 13) and the control group according to Test Example 3.
FIG. 7 is a picture showing the comparison of the cross-sectional areas of CNV lesions by performing a retinal imaging evaluation using OCT after administration of the composition of the present invention (Example 13) and the control group according to Test Example 3.
FIG. 8 is a graph showing the result of observing and digitizing the size of CNV lesions after performing a retinal imaging evaluation using FFA after administration of Example 19 among the compositions of the present invention, as well as Comparative Example 2 and the control group according to Test Example 4.

### Mode for Invention

Hereinafter, the present invention will be described in more detail through exemplary embodiments. These exemplary embodiments are provided only for the purpose of illustrating the present invention in more detail, and thus it will be apparent to those skilled in the art that the scope of the present invention is not limited thereto.

### Examples 1 to 9: Preparation of 0.1 w/v% to 0.5 w/v% APX-115 eye drops at pH 7.2

A composition containing 3-phenyl-4-propyl-1-(pyridin-2-yl)-1H-pyrazol-5-ol . hydrochloride (APX-115) was prepared with the addition of a solubilizing agent and a buffer agent as shown in table 1 below.

According to the following examples, the compositions were prepared by heating or adjusting pH depending on a concentration of the active ingredient, if necessary.

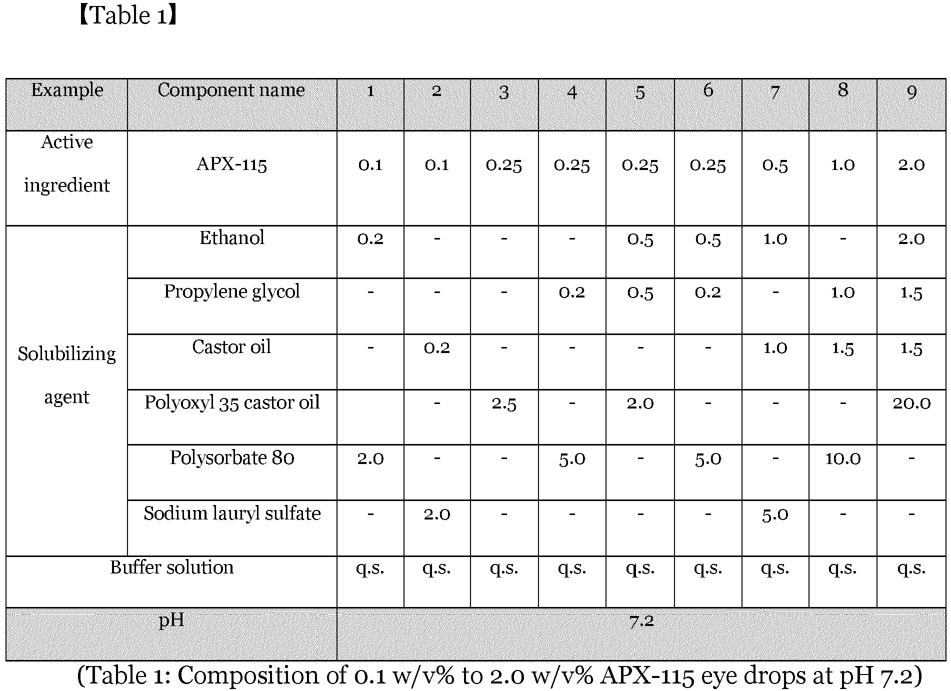

In Examples 1 to 9, anhydrous sodium dihydrogen phosphate and anhydrous sodium phosphate were used as the buffer solution, and the pH was adjusted with 0.5 N sodium hydroxide solution, if necessary.

Example 1: 0.2 g of ethanol and 2 g of polysorbate 80 were mixed and homogenized. After the resulting mixed solution was heated to 40°C or higher, 0.1 g of APX-115 was added and sonicated to dissolve and homogenize the resulting mixture, and then the buffer solution was added to adjust a final volume to 100 mL, and then filtered through a 0.22 um sterile filter.

Example 2: 0.1 g of APX-115 was added to 0.2 g of castor oil, heated to 40°C or higher, and sonicated to dissolve and homogenize the resulting mixture. A final volume was adjusted to 100 mL by using the buffer solution, in which 2 g of sodium lauryl sulfate was dissolved in the buffer solution of Example 1, to the mixed solution having APX-115 dissolved therein, and then filtered through a 0.22 µm sterile filter.

Example 3: 0.5 N sodium hydroxide solution was added to 25 mL of 10.0% polyoxyl 35 castor oil solution to adjust the pH to 9.0 or higher, and then 0.25 g of APX-115 was added to dissolve and homogenize the resulting mixture. A final volume was adjusted to 100 mL with the addition of the buffer solution to the mixed solution having APX-115 dissolved therein, and then filtered through a 0.22 µm sterile filter.

Example 4: 0.5 N sodium hydroxide solution was added to the mixed solution, in which 0.2 g of propylene glycol and 5 g of polysorbate 80 were mixed and homogenized, so as to adjust the pH to 9 or higher, and then 0.25 g of APX-115 was added to dissolve and homogenize the resulting mixture. A final volume was adjusted to 100 mL with the addition of the buffer solution to the mixed solution having APX-115 dissolved therein, and then filtered through a 0.22 µm sterile filter.

Examples 5 to 6: After homogeneously mixing each of the corresponding amounts of solubilizing agents specified in table 1, 0.25 g of APX-115 was added, heated to 40°C or higher, and mixed with shaking to dissolve and homogenize the resulting mixture. The buffer solution was added to the mixed solution to adjust a final volume to 100 mL, and then filtered through a 0.22 µm sterile filter.

Example 7: After homogeneously mixing 1 g of ethanol and 1 g of caster oil, 0.25 g of APX-115 was added, heated to 40°C or higher, and sonicated to dissolve and homogenize the resulting mixture. A final volume was adjusted to 100 mL by adding the buffer solution (5% sodium lauryl sulfate buffer solution), in which 5 g of sodium lauryl sulfate was dissolved in the buffer solution of Example 1, to the mixed solution having APX-115 dissolved therein, and then filtered through a 0.22 µm sterile filter.

Example 8: 0.5 N sodium hydroxide solution was added to the mixed solution, in which 1 g of propylene glycol and 1.5 g of caster oil were mixed and homogenized, so as to adjust the pH to 9 or higher, and then 1.0 g of APX-115 was added to dissolve and homogenize the resulting mixture. A final volume was adjusted to 100 mL by adding and mixing 10 g of polysorbate 80 to the mixed solution having APX-115 dissolved therein and adding the buffer solution, and then filtered through a 0.22 µm sterile filter.

Example 9: A mixed solution obtained by mixing and homogenizing 2 g of ethanol, 1.5 g of propylene glycol, and 1.5 g of castor oil was heated to 40°C or higher, after which 2.0 g of APX-115 was added to dissolve and homogenize the resulting mixture. A final volume was adjusted to 100 mL by adding and mixing 20 g of polyoxyl 35 castor oil to the mixed solution having APX-115 dissolved therein and adding the buffer solution, and then filtered through a 0.22 µm sterile filter.

### Examples 10 to 14: Preparation of 0.5 w/v% APX-115 eye drops at pH 3.5 to 8.5

Eye drops containing APX-115 and solubilizing agents and having different pHs were prepared according to table 2 below.

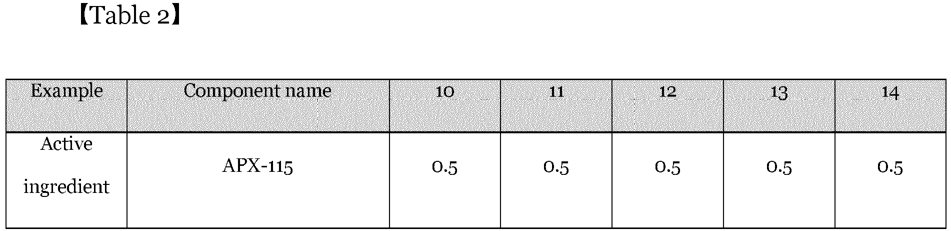

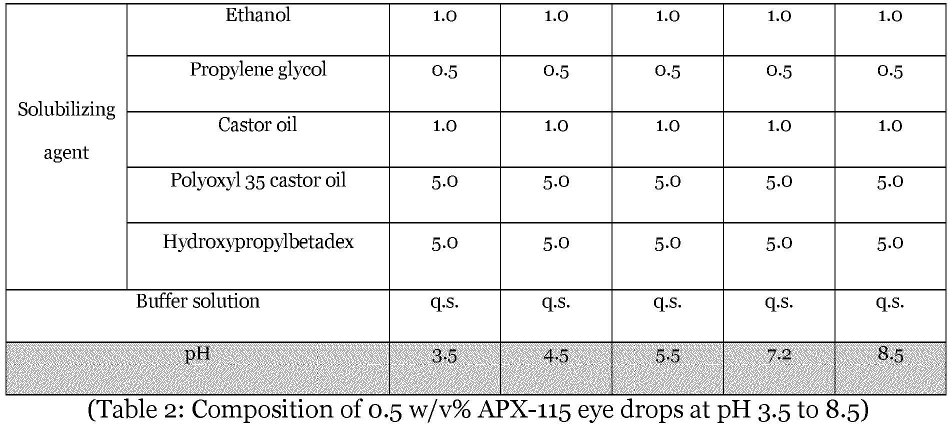

Examples 10 to 14: After weighing each of the corresponding amounts of solubilizing agents except hydroxypropylbetadex according to table 2 and homogeneously mixing the resulting solution, 0.5 g of APX-115 was added and mixed with shaking to dissolve and homogenize the resulting mixture. In order to prepare an eye drop adjusted to the corresponding pH, each buffer solution suitable for the pHs of Examples 10 to 14 was prepared, and then 5 g of hydroxypropylbetadex was added and dissolved. A volume was adjusted to 100 mL by adding the prepared buffer solution containing hydroxypropylbetadex to the mixed solution having APX-115 dissolved therein, and then filtered through a 0.22 µm sterile filter. In order to adjust pH suitable for each eye drop of Examples 10 to 14, the following buffer agent was used to prepare the buffer solution. Specifically, an ammonium acetate buffer solution at pH 3.5 was prepared with ammonium acetate and hydrochloric acid, an acetate buffer solution at pH 4.5 was prepared with acetic acid and sodium acetate, a citrate buffer solution at pH 5.5 was prepared with citric acid and disodium hydrogen phosphate, a phosphate buffer solution at pH 7.2 was prepared with sodium dihydrogen phosphate and sodium phosphate, and a borate buffer solution at pH 8.5 was prepared with borax.

### Examples 15 to 22: Preparation of 1.0 w/v% or 0.5 w/v% APX-115 eye drops at pH 4.5 to 8.5

Eye drops containing APX-115 and solubilizing agents and having different pHs were prepared according to table 3 below.

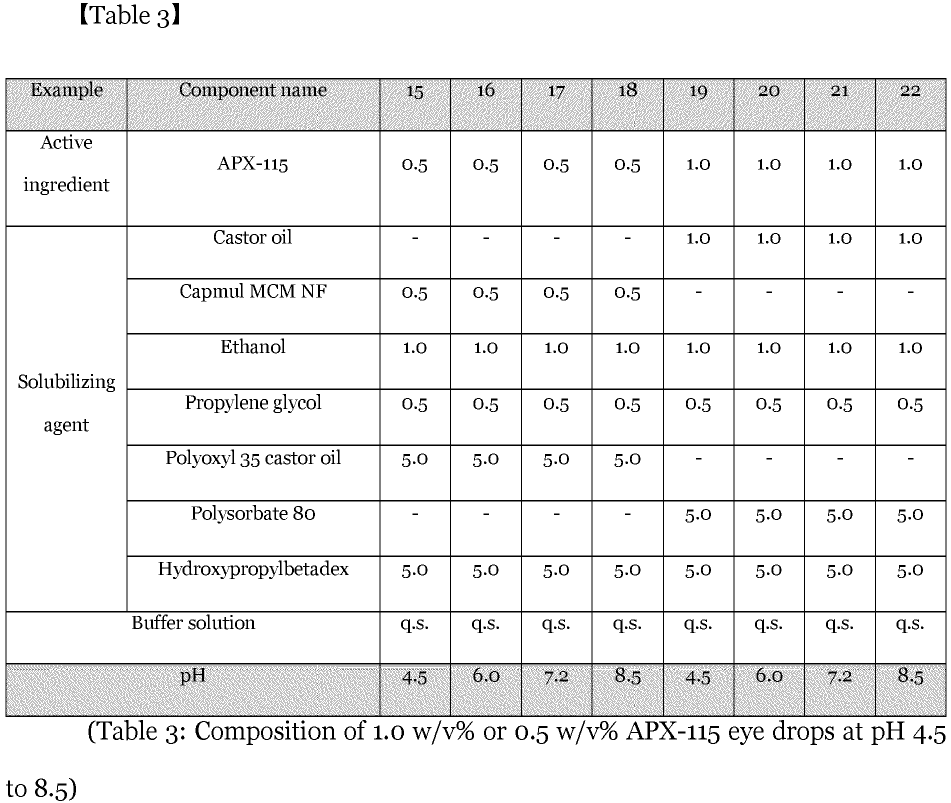

After weighing each of the corresponding amounts of solubilizing agents except hydroxypropylbetadex according to table 3 and homogeneously mixing the resulting solution, 0.5 g of APX-115 was added and mixed with shaking to dissolve and homogenize the resulting mixture. In order to prepare an eye drop adjusted to the corresponding pH, each buffer solution suitable for the pHs of Examples 15 to 22 was prepared, and then 5 g of hydroxypropylbetadex was added and dissolved. A volume was adjusted to 100 mL by adding the buffer solution containing hydroxypropylbetadex prepared in accordance with each pH to the solution having APX-115 dissolved therein, and then filtered through a 0.22 µm sterile filter. The buffer solutions at pH 4.5, 7.2, and 8.5 used were prepared by the same method as the buffer solutions used in Examples 10 to 14, and the buffer solution at pH 6.0 was prepared with disodium hydrogen phosphate and citric acid (pH adjusted with phosphoric acid).

### Examples 23 to 31: Preparation of 0.5 w/v% APX-115 eye drops at pH 7.2

The 0.5 w/v% APX-115 eye drop at pH 7.2 containing APX-115 and solubilizing agents was prepared according to table 4 below.

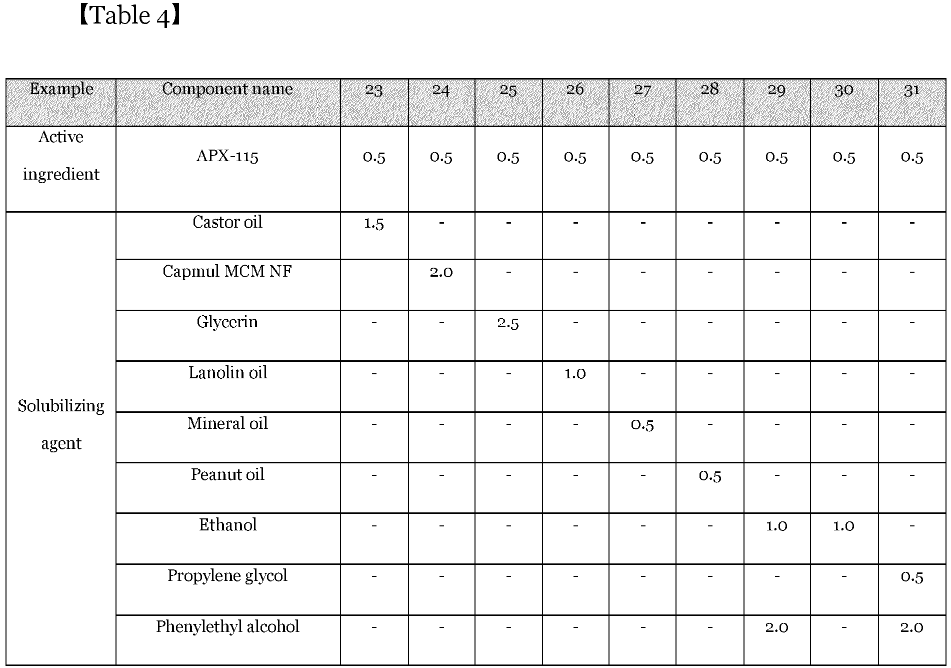

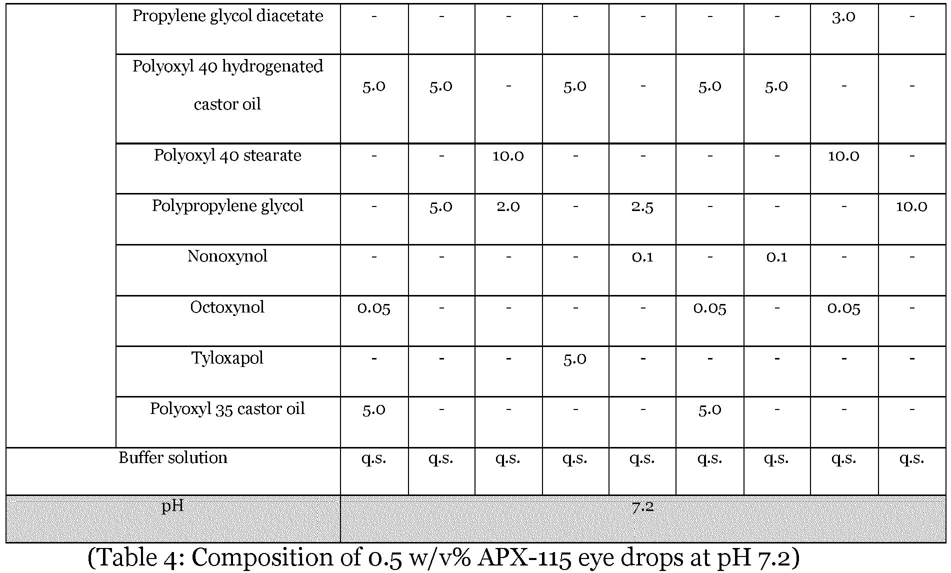

The solubilizing agents corresponding to each of Examples 23 to 31 were homogeneously dissolved and mixed, and then heated (40°C or higher). After adding 500 mg of APX-115 to each composition in which the solubilizing agents were mixed, the resulting mixture was sufficiently dissolved by stirring, and then a final volume was adjusted to 100 mL with a buffer solution at pH 7.2 so as to prepare 0.5 w/v% APX-115 eye drops. The buffer solution at pH 7.2 used was prepared by the same method as the buffer solution used in the preparation of Examples 1 to 9.

### Examples 32 to 40: Preparation of 0.5 w/v% APX-115 eye drops at pH 6.0

The 0.5 w/v% APX-115 eye drop at pH 6.0 containing APX-115 and solubilizing agents was prepared according to table 5 below.

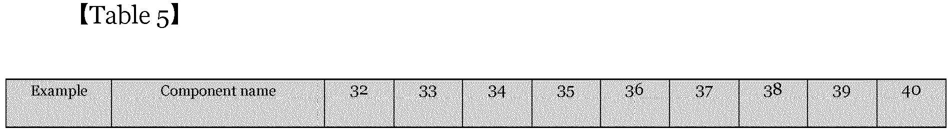

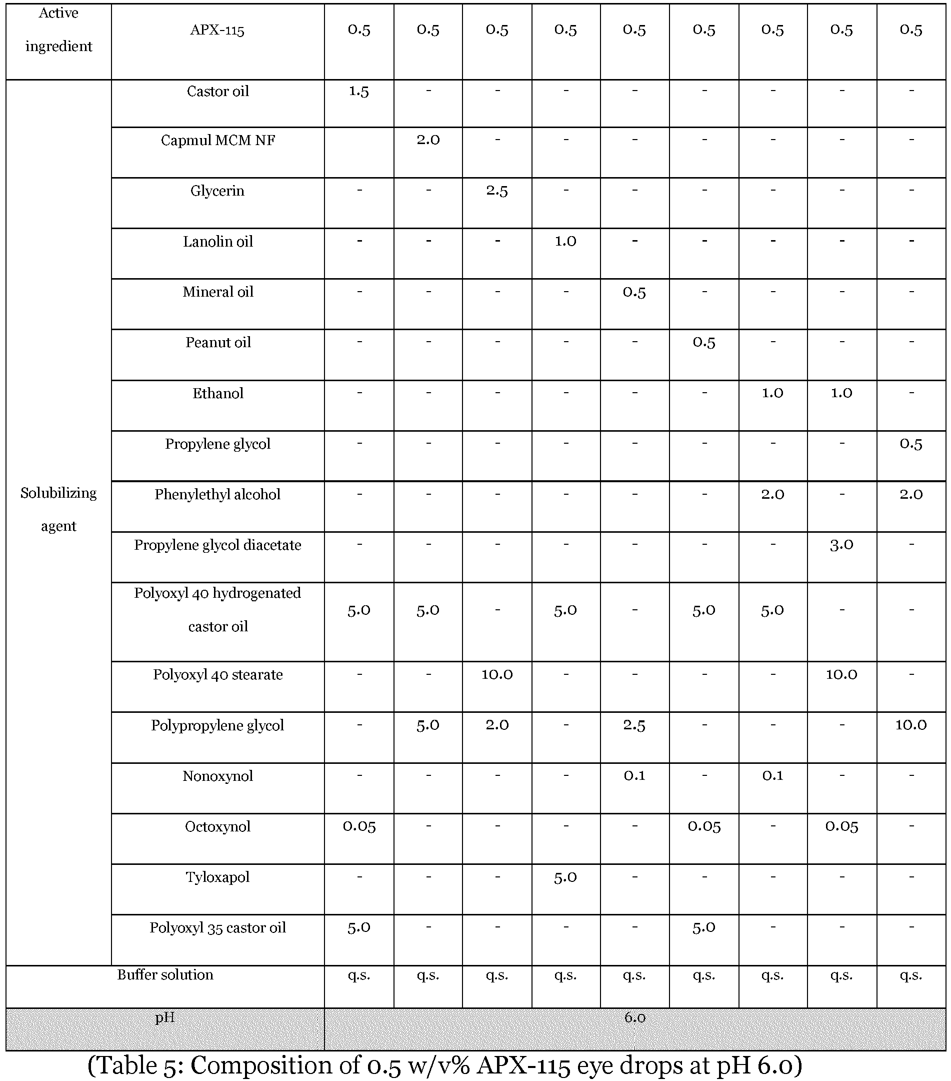

After adding 500 mg of APX-115 to a solution at pH 9 or higher (sodium hydroxide solution), the resulting mixture was sufficiently stirred and dissolved to adjust a final volume to 100 mL with a buffer solution containing additives according to the composition described in table 5 below, so as to prepare 0.5 w/v% APX-115 eye drops. The buffer solution at pH 6.0 was prepared with disodium hydrogen phosphate and citric acid, and the buffer solution used was the same as the buffer solutions used in the preparation of Examples 16 and 20.

### Comparative Examples 1 to 8: Preparation of APX-115 eye drops at pH 3.0 or lower or 9.0 or higher

Compositions containing APX-115 were prepared as shown in table 6 below so that the compositions have the same ingredients as and different pHs from those of Examples 10 to 22.

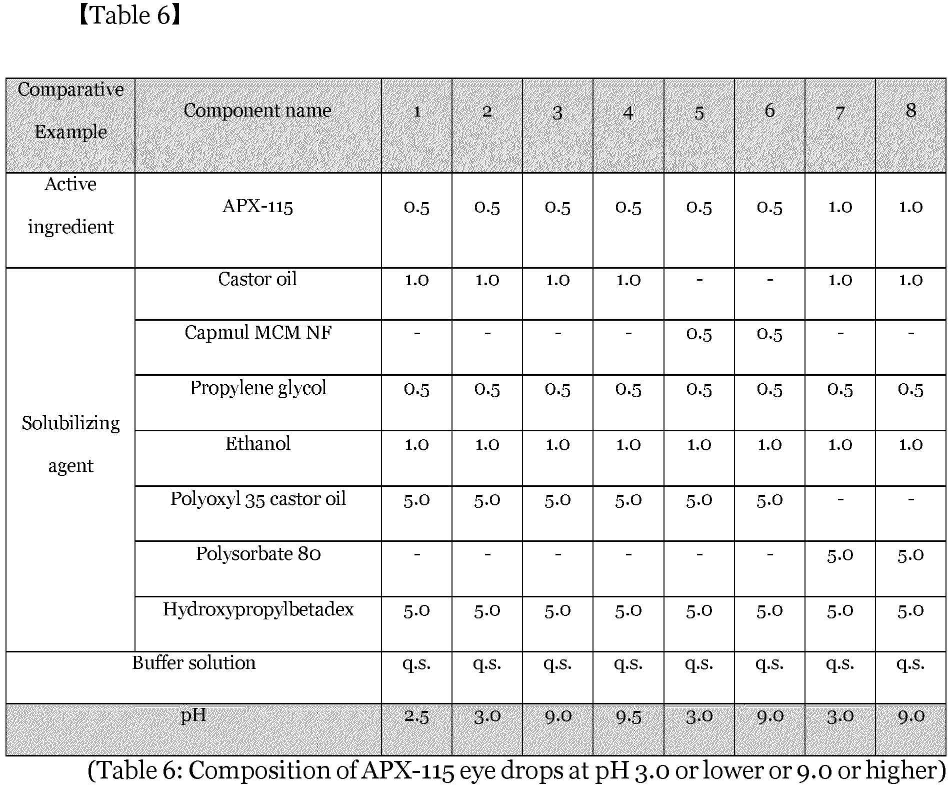

After weighing each of the corresponding amounts of solubilizing agents except hydroxypropylbetadex according to table 6 and homogeneously mixing the resulting solution, 0.5 g of APX-115 was added and mixed with shaking to dissolve and homogenize the resulting mixture. In order to prepare eye drops adjusted to the corresponding pHs, each buffer solution suitable for the pHs of Comparative Examples 1 to 8 was prepared, and then 5 g of hydroxypropylbetadex was added and dissolved. The buffer solution prepared in accordance with each pH was added to the solution in which APX-115 was dissolved, adjusted to a volume of 100 mL, and then filtered through a 0.22 µm sterile filter. Ammonium dihydrogen phosphate and phosphoric acid were used for the buffer solutions at pH 2.5 and pH 3.0, the buffer solution at pH 9.0 was prepared with sodium tetraborate and hydrochloric acid, and the buffer solution at pH 9.5 was prepared with 2-amino-2-hydroxymethyl-1,3-propanediol and hydrochloric acid.

### Test Example 1: Related Substance Test

The solutions prepared in above Examples and Comparative Examples were stored under the condition of 40°C/75% RH. In four weeks later, the maximum amount of unknown related substances among APX-115 decomposition products was measured by an HPLC method under the conditions described below.

### <Analysis Conditions>

- Column: Kromasil C18 (4.6 × 150 mm, 5 *µ*m)
- Column temperature: 30°C
- Mobile phase: 20mM ammonium formate (pH 3.0)/acetonitrile = 20/80 (v/v)
- Wavelength: 293 nm
- Injection amount: 10 µl

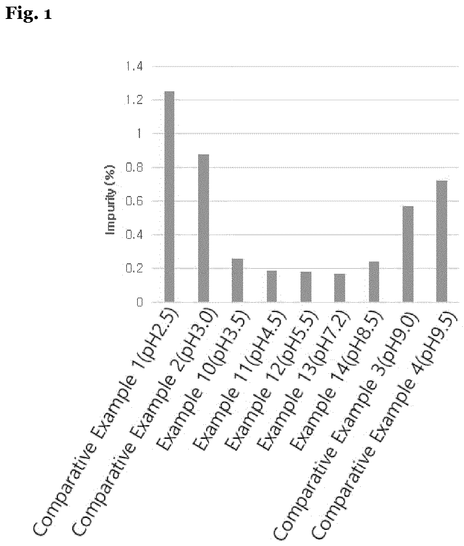

In the case of Examples 10 to 14 having the same composition of additives, but having pH ranges of 3.5 to 8.5 merely different from each other, as confirmed from FIG. 1, it can be understood that the related substances originating from the main ingredient are effectively suppressed, thereby improving the chemical stability of 3-phenyl-4-propyl-1-(pyridin-2-yl)-1H-pyrazol-5-ol compound. In contrast, in the case of Comparative Examples 1 and 2 having the same composition of additives, but having a pH of 3.0 or less, and in the case of Comparative Examples 3 and 4 having the same composition of additives, but having a pH of 9.0 or more, it was confirmed that 0.5% or more of impurities are generated within four weeks of storage after preparation, thereby not improving the chemical stability.

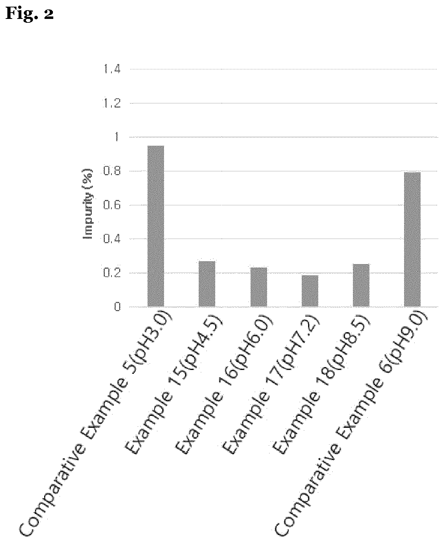

In addition, in the case of Examples 15 to 18 having the same composition of additives, but having pH ranges of 3.5 to 8.5 merely different from each other, as confirmed from FIG. 2, it seems that the 3-phenyl-4-propyl-1-(pyridin-2-yl)-1H-pyrazol-5-ol compound shows an improvement in stability. In the case of Comparative Examples 5 (pH 3.0) and 6 (pH 9.0), it seems that the compositions thereof show stability lower than that of the compositions of Examples.

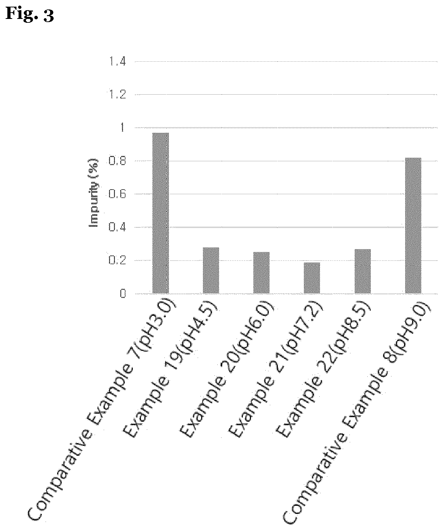

Furthermore, in the case of Examples 19 to 22 having a pH range of 3.5 to 8.5, as confirmed from FIG. 3, it seems that the 3-phenyl-4-propyl-1-(pyridin-2-yl)-1H-pyrazol-5-ol compound shows an improvement in stability with less generation of impurities. In the case of Comparative Examples 7 (pH 3.0) and 8 (pH 9.0), it seems that the compositions thereof show stability lower than that of the compositions of Examples.

In other words, it was confirmed from the results of Test Example 1 that the eye drop of one embodiment containing the 3-phenyl-4-propyl-1-(pyridin-2-yl)-1H-pyrazol-5-ol compound shows excellent stability in the pH range of 3.5 to 8.5.

According to the ICH guidelines, for a drug product with a maximum daily dose of 1 mg or more and 10 mg or less, a criterion for managing unknown impurities with an unidentified structure is set to 0.5% or less. Accordingly, it was confirmed that the compositions of Examples 10 to 22 having a pH range of 3.5 to 8.5 show a remarkable improvement in the stability of the 3-phenyl-4-propyl-1-(pyridin-2-yl)-1H-pyrazol-5-ol compound compared to the compositions of Comparative Examples 1 to 8 having a pH range of 3.0 or lower or 9.0 or higher.

### Test Example 2: Test on eye mucosal irritation

After instilling 50 uL of each of the compositions of above Examples 10 to 14 and Comparative Examples 1 to 4 once into both eyes of a New Zealand white rabbit weighing 2.0 to 2.7 kg, the behavioral patterns of the experimental animal were observed. Examples 10 to 14 were used as a test group, Comparative Examples 1 to 4 were used as a Comparative group, and Diquas^{®}, which is a commercially available eye drop, was used as a positive control group. Immediately after drug administration, the behavioral patterns of each rabbit were observed and shown in the table below.

### [Table 7]

**(Table 7: Results of eye mucosal irritation test evaluation)**

| Example | pH | Irri int tation ensity | Comparative Example | pH | Ir in rrit te ation nsity^{∗} |
|---|---|---|---|---|---|
| 10 | 3.5 | +(+) | 1 | 2.5 | +++ |
| 11 | 4.5 | + | 2 | 3.0 | +++ |
| 12 | 5.5 | + | 3 | 9.0 | ++ |
| 13 | 7.2 | + | 4 | 9.5 | +++ |
| 14 | 8.5 | + | Diquas^{®} | 7.3 | + |

| | | | | | |
|---|---|---|---|---|---|
| Irritation intensity^{∗} +++: Very serious, ++: Moderate, +: Mild, +(+): More serious than + and weaker than ++ | | | | | |

As a result of evaluating an eye mucosal irritation using rabbits, Examples 10 to 14, which are compositions of the present invention, showed a degree of reaction (eye blinking) similar to that of Diquas^{®} available on the market unlike Comparative Examples 1 to 4 with severe irritation intensity, and thus it was confirmed that the composition of the present invention having the pH 3.5 to 8.5 shows good results even for eye mucosal irritation. In particular, it was confirmed that the composition at pH 4.5 to 8.5 shows a more excellent degree of eye mucosal irritation.

### Test Example 3. Test on intraocular tissue distribution (intravitreal PK evaluation)

In order to confirm the effect of the composition of the present invention in which a drug thereof is delivered to the posterior segment through ophthalmic administration alone without injection administration, thereby showing a therapeutic efficacy, a concentration of the active ingredient in the vitreous body adjacent to the retina/choroid among the posterior segment tissues of the eyeball was confirmed through the test of Test Example 3. In addition, it was confirmed from a previous study on stability (Test Example 1) that there is a difference in stability of the composition depending on the pH of the composition, and a test on PK distribution for each pH was performed in order to evaluate an amount of the active ingredient delivered to the posterior segment tissues depending on pH of the composition, since the degree of ionization of the active ingredient varies according to the pH.

The evaluation method of this Test Example 3 is an efficient evaluation method capable of predicting the efficacy on posterior segment eye diseases by measuring the amount of the drug in the vitreous body inside the eyeball, which is delivered by instillation, and thus by comparing the amount of the drug that can reach the posterior segment, which is the target organ.

With regard to the compositions of Examples prepared to have the pH range of 3.5 to 8.5 and the compositions of Comparative Examples prepared to have the pH range of 3.5 or lower or 9.5 or higher among the compositions of the present invention, each of 40 uL of those compositions was instilled once into both eyes of a New Zealand white rabbit weighing 2.0 to 2.7 kg. In 30 minutes later, a vitreous humor was collected from the eye tissues of the experimental animal and the concentration of the compound in the sample was measured by using an LC/MS/MS equipment.

### <Analysis Conditions >

- Column: Waters Acquity UPLC BEH C18 (50 × 2.1 mm, 1.7 mm)
- Column temperature: 40°C
- Mobile phase: Acetonitrile/0.1% formic acid = 70/30 (v/v)
- Injection amount: 5 µl
- Flux: 0.2 mL/min

### [Table 8]

**(Table 8: Concentration of active ingredient in vitreous humor after instillation of the composition of the present invention)**

| Composition (concentration of active ingredient in eye drops) | pH | Concentration of active ingredient in vitreous humor (ng/mL) |
|---|---|---|
| Comparative Example 2 (0.5 w/v%) | 3.0 | 0.8 ± 0.5 |
| Example 10 (0.5 w/v%) | 3.5 | 2.5 ± 1.0 |
| Example 11 (0.5 w/v%) | 4.5 | 4.8 ± 3.2 |
| Example 16 (0.5 w/v%) | 6.0 | 5.1 ± 3.4 |
| Example 34 (0.5 w/v%) | 6.0 | 4.3 ± 2.7 |
| Example 1 (0.1 w/v%) | 7.2 | 3.1 ± 1.6 |
| Example 3 (0.25 w/v%) | 7.2 | 4.0 ± 3.1 |
| Example 13 (0.5 w/v%) | 7.2 | 5.7 ± 2.9 |
| Example 14 (0.5 w/v%) | 8.5 | 3.5 ± 1.9 |
| Comparative Example 4 (0.5 w/v%) | 9.5 | 0.3 ± 0.2 |

As a result of the eyeball PK test using rabbits, it was observed that the concentration of the active ingredient in the vitreous humor is very low in Comparative Example 2 at pH 3.0 and Comparative Example 4 at pH 9.5 and thus it could be confirmed that the amount of the active ingredient delivered to the posterior segment after instillation is very small. In contrast, it was observed that the concentration of the active ingredient in the vitreous humor is remarkably high in Examples 1, 3, 10, 11, 13, 14, 16 and 34 at pH 3.5 to 8.5 compared to Comparative Examples. In particular, the 3-phenyl-4-propyl-1-(pyridin-2-yl)-1H-pyrazol-5-ol compound has a maximum level of lipophilicity (hydrophobicity) from pH 5 or lower due to the properties of the drug. Thus, although all the biomembrane permeabilities were theoretically the same in an acidic state of pH 5 or less, it was confirmed that the delivery of the active ingredient to the posterior segment becomes very low, particularly at pH 3.0 or less.

From the above results, it was confirmed that the delivery power of the 3-phenyl-4-propyl-1-(pyridin-2-yl)-1H-pyrazol-5-ol compound to the posterior segment after instillation is most excellent in the pH range of 3.5 to 8.5.

In addition, with regard to the ophthalmic composition of one embodiment, the concentration of the active ingredient in the vitreous humor shows the same degree of concentration even when using different solubilizing agents. Thus, it was confirmed that the solubilizing agents do not have a significant effect on the delivery of the active ingredient to the posterior segment, as long as the solubilizing agents are capable of solubilizing the active ingredient herein.

### Test Example 4. Evaluation of validity of ophthalmic composition using mouse CNV model

It was confirmed from the previous test on distribution inside eyeball tissues (Test Example 3) that the active ingredient is distributed to the vitreous body adjacent to the retina, which is the posterior segment tissue, when administering the ophthalmic composition for each pH according to one embodiment. After that, in order to confirm the inhibitory effect of the ophthalmic composition of the present invention (Example 13) on choroidal neovascularization (CNV), the efficacy was evaluated with the mouse CNV model by selecting the composition of the present invention as a test material, a placebo material as a negative control material (Gi), and Eylea^{®}, which is a commercially available injection, as a positive control material (G2).

The negative control material (Gi) and test materials (G₃ and G₄) were instilled at a dose of 5 uL once from the day after CNV induction, and the positive control material (G2) was directly injected once into the vitreous body at a dose of 1 uL (20 ug/uL) by using a 36 G syringe on the day after CNV induction. The negative control material was administered four times a day. The efficacy of the test material was compared and evaluated according to the number of daily administrations, which was divided into an administration group of four times (G₃) and an administration group of eight times (G₄).

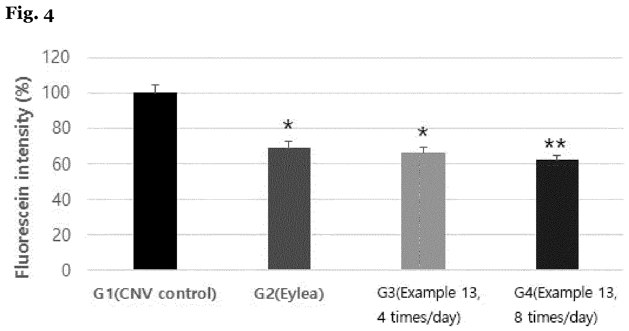

Specifically, on the 12th day after CNV induction, the experimental animal was subjected to general anesthesia and to intraperitoneal injection of a fluorescent contrast agent. Then, an anesthetic eye drop was applied into the eyeball of the experimental animal to induce additional local anesthesia. After that, mydriatic was instilled to induce mydriasis and perform a retinal image evaluation. Thus, the results are shown in FIGS. 4 to 7.

As a result of the test, as can be confirmed from FIGS. 4 to 7, in the case of administering the composition of the present invention four times only a day (G₃), the composition of the present invention showed an effect equal to or higher than that of the group dosed with Eylea^{®} (positive control group, G2), which is directly injected into the vitreous body.

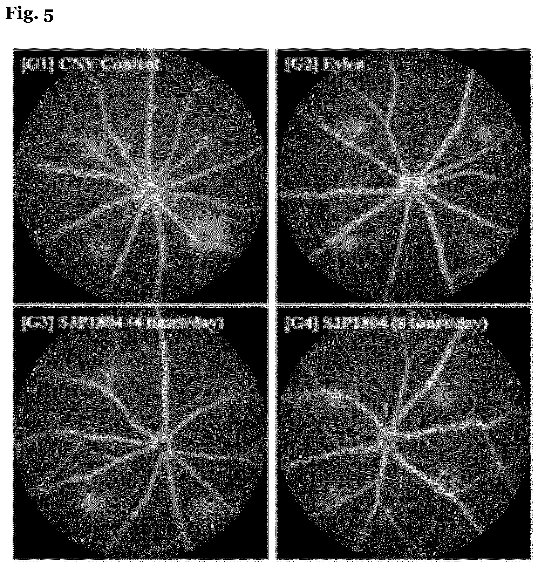

The results of the evaluation using fundus fluorescein angiography (FFA) during retinal imaging evaluation are shown in FIGS. 4 and 5. Referring to FIGS. 4 and 5, it was confirmed from the comparison between the negative control group (Gi) and the test groups (G₃ and G₄) that there is a significant difference therebetween and the size of the lesion is reduced in the test group, thereby providing a therapeutic effect. In addition, it was confirmed that administration of four times only (G₃) shows a similar therapeutic effect compared to Eylea, which is the positive control group (G2).

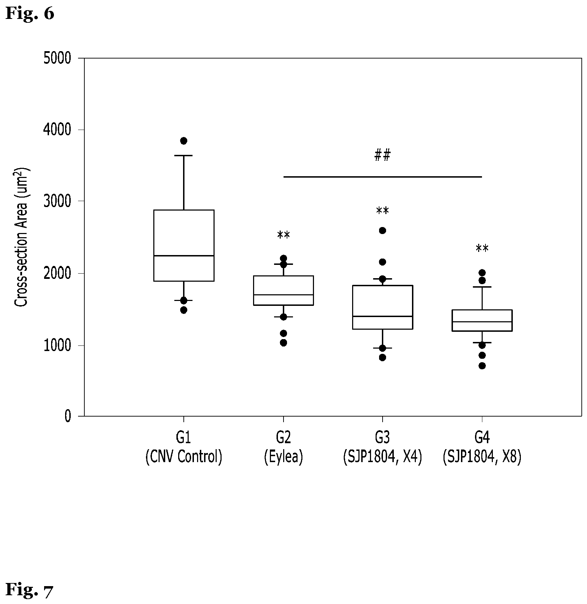

FIGS. 6 and 7 show the results of evaluation using an optical coherence tomography (OCT) during retinal imaging evaluation. Referring to FIGS. 6 and 7, it was confirmed that the groups dosed with the test material (G₃, G₄) show a therapeutic effect due to a decrease in the cross-sectional area of the lesion in the test groups, and also show a statistically significant difference even when comparing the negative control group with the test groups. In addition, it was confirmed that instillation of four times only a day (G₃) shows a therapeutic effect equal to or higher than that of the group dosed with Eylea, which is the positive control group (G2).

As a result of evaluating the efficacy of the composition of the present invention through Test Example 4, it was confirmed that the composition of the present invention has an effect equal to or higher than that of the commercially available Eylea injection (the positive control group, G2) in the CNV animal model.

### Test Example 5. Evaluation of validity according to pH change of ophthalmic composition

A validity evaluation was performed by the same method as Test Example 4 with each ophthalmic composition having the same composition, but having different pHs.

Among the ophthalmic compositions of the present invention, Example 19 (G₃) showed excellent stability and excellent PK results, and Comparative Example 2 (G₄) showed low stability and poor PK test results. Accordingly, to confirm the inhibitory effect of both Example 19 (G₃) and Comparative Example 2 (G₄) on choroidal neovascularization (CNV), Example 19 and Comparative Example 2 were selected as a test material out of the compositions of the present invention, a placebo material was selected as a negative control material (Gi), and Eylea^{®}, which is a commercially available injection, was selected as a positive control material (G2), thereby evaluating the efficacy of the mouse CNV model.

The negative control material (Gi) and test materials (G₃ and G₄) were instilled at a dose of 5 uL four times a day from the day after CNV induction, and the positive control material (G2) was directly injected once into the vitreous body at a dose of 1 uL (20 ug/uL) by using a 36 G syringe on the day after CNV induction.

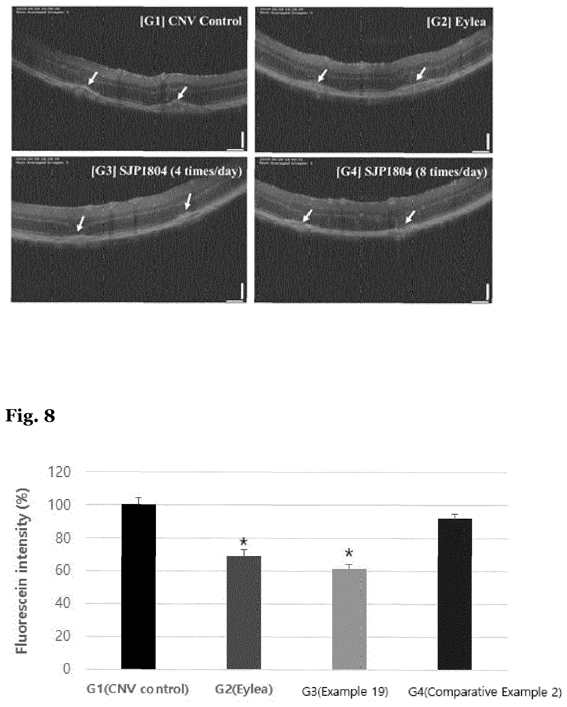

As a result of the test, as can be confirmed from FIG. 8 (evaluation using FFA), in the case of administering Example 19 four times only a day (G₃) out of the compositions of the present invention, it was confirmed that Example 19 shows an efficacy equal to or higher than that of the group dosed with Eylea^{®} (positive control group, G2), which is directly injected into the vitreous body, and shows a significant therapeutic effect compared to the group dosed with the negative control material. In contrast, Comparative Example 2 was found to have no significance compared to the group dosed with the negative control material, and thus it was confirmed that Comparative Example 2 has a low or almost no effect of inhibiting choroidal neovascularization.

It could be confirmed from Test Examples 1 to 5 of the present invention that the eye drop of the 3-phenyl-4-propyl-1-(pyridin-2-yl)-1H-pyrazole-5-ol compound containing a solubilizing agent and a buffer agent and maintaining the pH range of 3.5 to 8.5 secures stability and safety as a drug product and has a remarkable effect on delivering a drug to the posterior segment as well as a remarkable therapeutic effect.

## Claims

1. An eye drop for preventing or treating eye diseases, comprising: 3-phenyl-4-propyl-1-(pyridin-2-yl)-1H-pyrazol-5-ol represented by a following formula 1 or a pharmaceutically acceptable salt thereof as an active ingredient; a solubilizing agent; and a buffer agent, in which a pH range is 3.5 to 8.5:

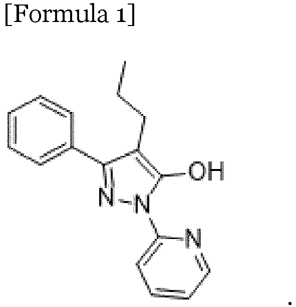

2. An eye drop for preventing or treating eye diseases, comprising: 3-phenyl-4-propyl-1-(pyridin-2-yl)-1H-pyrazol-5-ol represented by a following formula 1 or a pharmaceutically acceptable salt thereof by 0.1 w/v% to 2.0 w/v% as an active ingredient; a solubilizing agent; and a buffer agent, in which a pH range is 3.5 to 8.5:

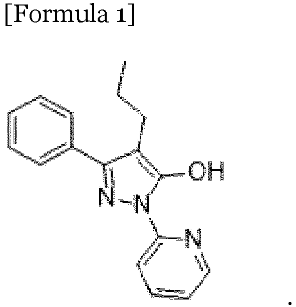

3. An eye drop for preventing or treating eye diseases, comprising: 3-phenyl-4-propyl-1-(pyridin-2-yl)-1H-pyrazol-5-ol represented by a following formula 1 or a pharmaceutically acceptable salt thereof by 0.1 w/v% to 2.0 w/v% as an active ingredient; a solubilizing agent; and a buffer agent, in which a pH range is 4.5 to 8.5:

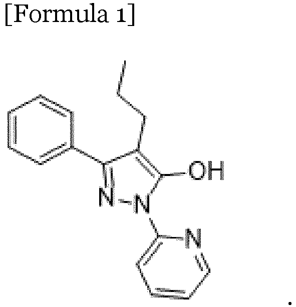

4. The eye drop according to any one of claims 1 to 3, wherein the pharmaceutically acceptable salt is hydrochloride.

5. The eye drop according to any one of claims 1 to 3, wherein a concentration of the solubilizing agent is 0.1 w/v% to 25.0 w/v%.

6. The eye drop according to any one of claims 1 to 3, wherein the solubilizing agent is at least one selected from the group consisting of surfactants, solvents, and mixtures thereof.

7. The eye drop according to any one of claims 1 to 3, wherein the solubilizing agent comprises at least one of surfactants.

8. The eye drop according to any one of claims 1 to 3, wherein the eye disease is a posterior segment eye disease.

9. The eye drop according to claim 8, wherein the posterior segment eye disease is at least one selected from the group consisting of diabetic retinopathy (DR), diabetic macular edema, age-related macular degeneration, acute macular neuroretinopathy, retinopathy of prematurity (ROP), polypoidal choroidal vasculopathy, ischemic proliferative retinopathy, retinitis pigmentosa, cone dystrophy, Behcet's disease, retinal disorders, proliferative vitreoretinopathy (PVR), retinal artery occlusion, retinal vein occlusion, retinitis, uveitis, Leber's hereditary optic neuropathy, retinal detachment, retinal pigment epithelial detachment, neovascular glaucoma, retinal neovascularization and choroidal neovascularization (CNV), trauma of the posterior segment, radiation retinopathy, epiretinal membrane disorder, branch retinal vein occlusion, anterior ischemic optic nerve disorder, non-retinopathy diabetic retinal dysfunction, retinitis pigmentosa, and glaucoma.

10. A method for preventing or treating eye diseases, the method comprising: administering an eye drop containing 3-phenyl-4-propyl-1-(pyridin-2-yl)-1H-pyrazol-5-ol represented by a following formula 1 or a pharmaceutically acceptable salt thereof as an active ingredient; a solubilizing agent; and a buffer agent and also having a pH range of 3.5 to 8.5 into a subject in need of treatment:

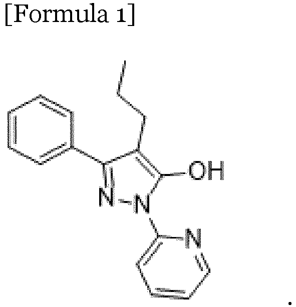

11. A use of an eye drop comprising 3-phenyl-4-propyl-1-(pyridin-2-yl)-1H-pyrazol-5-ol represented by a following formula 1 or a pharmaceutically acceptable salt thereof as an active ingredient; a solubilizing agent; and a buffer agent and also having a pH range of 3.5 to 8.5 for preventing or treating eye diseases:

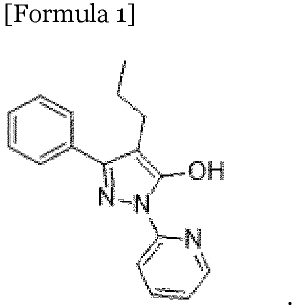

12. A use of an ophthalmic composition comprising 3-phenyl-4-propyl-1-(pyridin-2-yl)-1H-pyrazol-5-ol represented by a following formula 1 or a pharmaceutically acceptable salt thereof as an active ingredient; a solubilizing agent; and a buffer agent and also having a pH range of 3.5 to 8.5, in preparation of an eye drop for preventing or treating eye diseases:
